(19) Europäisches Patentamt
European Patent Office
Office européen des brevets

(11) EP 1 488 789 A1

(12) EUROPEAN PATENT APPLICATION
published in accordance with Art. 158(3) EPC

(43) Date of publication:
22.12.2004 Bulletin 2004/52

(21) Application number: 03744029.4

(22) Date of filing: 10.03.2003

(51) Int Cl.7: **A61K 31/41**, A61K 31/4178, A61K 31/4184, A61K 31/437, A61K 31/4439, A61K 47/18, A61P 27/02, C07D 257/04, C07D 401/10, C07D 403/10, C07D 471/04

(86) International application number:
PCT/JP2003/002771

(87) International publication number:
WO 2003/075920 (18.09.2003 Gazette 2003/38)

(84) Designated Contracting States:
AT BE BG CH CY CZ DE DK EE ES FI FR GB GR HU IE IT LI LU MC NL PT RO SE SI SK TR
Designated Extension States:
AL LT LV MK

(30) Priority: 08.03.2002 JP 2002063260

(71) Applicant: Sankyo Company, Limited
Tokyo 103-8426 (JP)

(72) Inventors:
- UNEDA, Takeshi,
c/o SANTEN PHARMACEUTICAL CO., LTD
Osaka-shi, Osaka 533-8651 (JP)
- KIMURA, Akio,
c/o SANTEN PHARMACEUTICAL CO., LTD
Osaka-shi, Osaka 533-8651 (JP)
- MORISHIMA, Kenji,
c/o SANTEN PHARMACEUTICAL CO LTD
O saka-shi, Osaka 533-8651 (JP)

(74) Representative:
TER MEER STEINMEISTER & PARTNER GbR
Patentanwälte,
Mauerkircherstrasse 45
81679 München (DE)

# (54) EYE DROPS CONTAINING TETRAZOLE DERIVATIVE

(57) The purpose of the invention is to find out additives which enhance the antiseptic effect of a cationic antiseptic agent added to ophthalmic solutions containing a compound having the chemical structure of the following general formula (I). The antimicrobial effect of the ophthalmic solutions containing a compound having the chemical structure of the general formula (I) and a cationic antiseptic agent is markedly increased by adding a basic amine compound to the solutions. In the general formula (I), $R^1$ is a group of the following structural formula (Ia), (Ib), (Ic), (Id), (Ie) or (If).

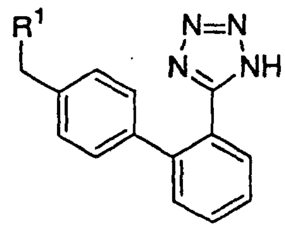

(I)

EP 1 488 789 A1

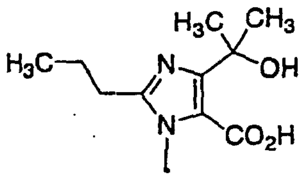

(Ia)

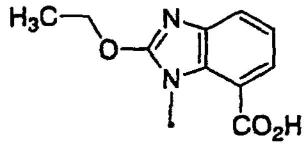

(Ib)

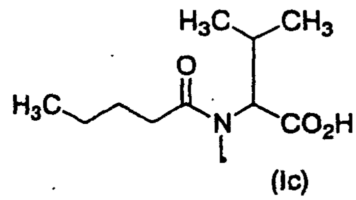

(Ic)

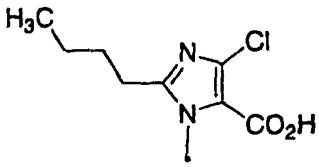

(Id)

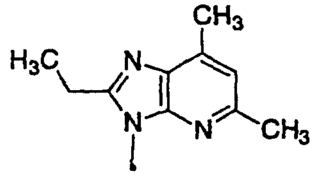

(Ie)

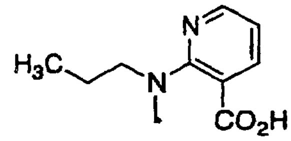

(If)

## Description

Technical Field

The present invention relates to highly antiseptic ophthalmic solutions containing an intraocular pressure reducing agent as well as to ophthalmic solutions in which an intraocular pressure reducing agent is dispersed in a molecular state in a vehicle for ophthalmic solutions.

Background Art

It has so far been known that angiotensin II antagonists decrease intraocular pressure on local administration (EP-A795326; EP-A631780; WO 95/21609; WO 91/15206 ; and so on). The following compounds are known as typical examples of the antagonists:

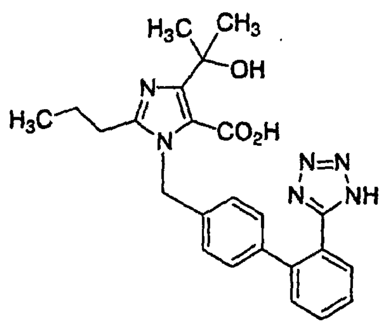

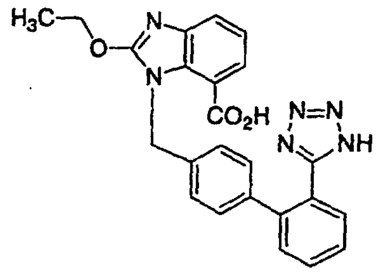

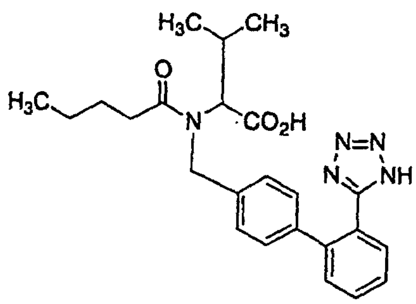

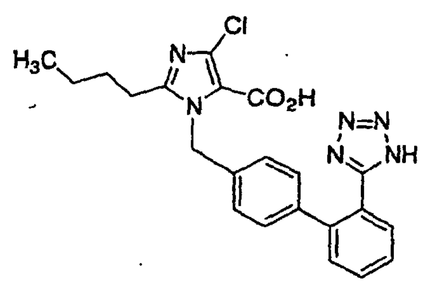

JP-A 2001-55329 discloses a locally administerable composition decreasing intraocular pressure in which boric acids and ethylenediaminetetraacetic acids in combination are formulated in an ophthalmic solution containing angiotensin II antagonist in order to enhance the intraocular pressure reducing effect of the antagonist.

On the other hand, selection of formulation components should be made carefully particularly in ophthalmic solutions because they are directly administered to eyes, highly sensitive organs of the human body. Antiseptic agents for use in ophthalmic solutions cannot be considered similarly as those (preservatives) used in foods, drinks, preservatives for contact lens, and the like. For example, it is necessary to consider the irritant properties of the ophthalmic solutions to eyes, various side effects occurring after administration to eyes, and the time-depending stability of the ophthalmic solutions permitting long-term preservation.

The antiseptic agents so far employed in ophthalmic solutions include benzalkonium chloride, benzethonium chloride, parahyroxybenzoic acid esters, chlorobutanol, chlorhexidine, sorbic acid salts, and the like. In particular, among those antiseptics, cationic one such as benzalkonium chloride or chlorhexidine has widely been used since they are chemically stable and have a good antiseptic effect. However, even if a cationic antiseptic agent is formulated in the ophthalmic solutions containing a compound which has the chemical structure of the following general formula (I), it would give no desired preservative effect and sometimes make the ophthalmic solutions turbid.

The compound having the chemical structure of the following general formula (I) has the properties of forming such an associated molecule (micell) as tetramer to octamer in an aqueous solution depending on the concentration, and sometimes forms a heterogeneous solution microscopically.

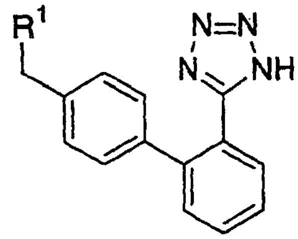

(I)

[wherein $R^1$ represents a group of the following structural formula (Ia), (Ib), (Ic), (Id), (Ie) or (If)]

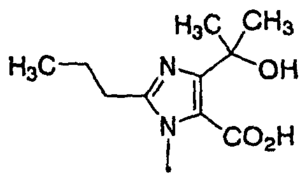

(Ia)

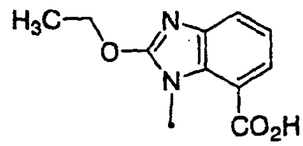

(Ib)

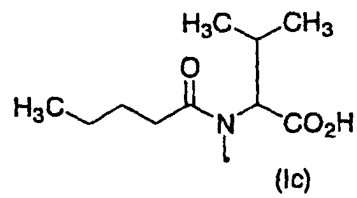

(Ic)

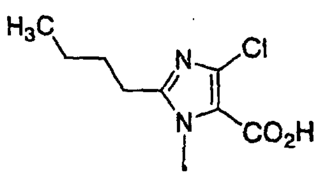

(Id)

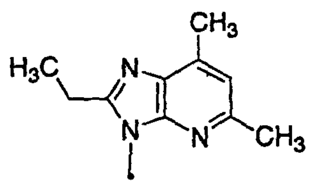

(Ie)

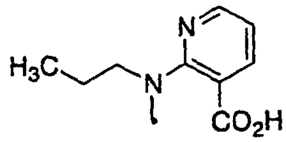

(If)

In this situation, when a cationic antiseptic agent is formulated in ophthalmic solutions containing a compound of the general formula (I), additives to enhance the antiseptic effect of the cationic agent with no turbidity in the ophthalmic solutions have been required. It has also been required that the compound which has the chemical structure of the general formula (I) causes no molecular association in the ophthalmic solutions to yield highly microscopically homogeneous ophthalmic solutions.

Disclosure of Invention

In order to solve the above-mentioned problems, the present inventors worked intensively to study additives to ophthalmic solutions and found that when a basic amine compound such as trometamol [tris(hydroxymethyl)aminomethane], lidocaine, or nicotinamide is formulated in the ophthalmic solutions containing a compound of the general formula (I) and a cationic antiseptice agent , such a basic amine compound markedly enhances the antiseptic effect of the ophthalmic solutions and yield no turbidity in the ophthalmic solutions. Thus, the invention was completed.

That is, the present invention provides (1) a highly antiseptic ophthalmic solution which comprises a compound having the chemical structure of the general formula (I) or its pharmaceutically acceptable salt or derivative (hereinafter referred to as "the present compound"), a cationic antiseptic agent and a basic amine compound.

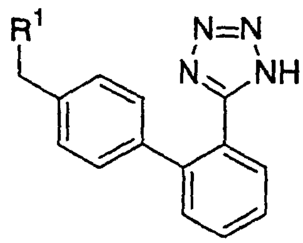

(I)

[wherein $R^1$ represents a group of the following structural formula (Ia), (Ib), (Ic), (Id), (Ie) or (If)]

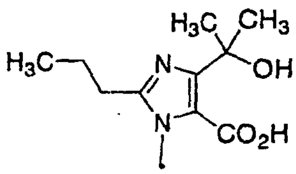

(Ia)

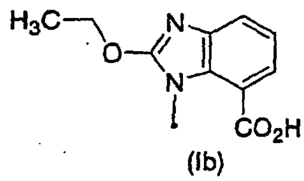

(Ib)

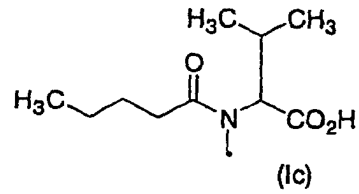

(Ic)

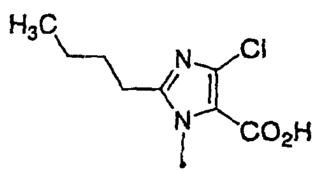

(Id)

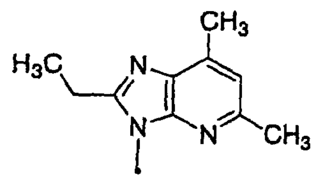

(Ie)

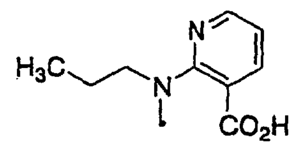

(If)

In the above-mentioned ophthalmic solutions, the present invention preferably provides:

(2) the ophthalmic solution wherein $R^1$ is a group of the above-mentioned structural formula (Ia), (Ib) or (Ic);
(3) the ophthalmic solution wherein the present compound is selected from:

4-(1-hydroxy-1-methylethyl)-2-propyl-1-{4-[2-(tetraz ol-5-yl)phenyl]phenyl}methylimidazole-5-carboxylic acid and
2-ethoxy-1-[2'-(1H-tetrazol-5-yl)biphenyl-4-yl]methy 1]-1H-benzimidazole-7-carboxylic acid;

(4) the ophthalmic solution wherein the cationic antiseptic agent is a quaternary ammonium salt;
(5) the ophthalmic solution wherein the quaternary ammonium salt is benzalkonium chloride or cetrimide;
(6) the ophthalmic solution wherein the basic amine compound is at least one selected from trometamol, lidocaine and nicotinamide;
(7) the ophthalmic solution additionally containing boric acids;
(8) the ophthalmic solution additionally containing ethylenediaminetetraacetic acids; and
(9) the ophthalmic solution additionally containing boric acids and ethylenediaminetetraacetic acids.

Subsequently, the ophthalmic solution of the present invention was analyzed by means of NMR to determine the chemical shift of each proton in the present compound, and found that the chemical shift of each proton in the ophthalmic solution of the present invention appears at a higher magnetic field (upfield) compared with that in an aqueous solution of the present compound. The shift of the chemical shift to the higher magnetic field indicates that the electron density of each proton of the present compound is changed and that the molecular association of the present compound is dissociated in the ophthalmic solution resulting in dispersion almost in a state of the molecule. In addition, the measured particle size of the associated molecule (micell) of the present compound was found to be

reduced to 1/2 or less. That is, in another aspect, the present invention provides an ophthalmic solution containing the present compound as an active ingredient wherein the active ingredient is dispersed in a molecular state in a vehicle for ophthalmic solutions.

In the invention, the "cationic antiseptic agent" means those which are positively charged in an aqueous solution, including, for example, a quaternary ammonium salt, chlorhexidine, and the like, preferably quaternary ammonium salt. The quaternary ammonium salt includes, for example, benzalkonium chloride, benzethonium chloride, cetylpyridinium chloride, cetrimide, and the like, preferably benzalkonium chloride and cetrimide. The concentration of the cationic antiseptic agent in the ophthalmic solution is preferably 0.0001 to 1.0% (w/v), more preferably 0.001 to 0.5% (w/v), but not limited thereto.

The above-mentioned cationic antiseptic agents may be used alone or in combination of two or more members.

In addition to the above-mentioned cationic antiseptic agents, other antiseptic agents such as sorbic acid, potassium sorbate, parahydroxybenzoic acid esters, chlorobutanol, benzyl alcohol, thimerosal, and the like may be used in combination.

In the present invention, the "basic amine compound" means an amine having no acidic group such as carboxylic acid in the molecule and showing a basicity as the whole molecule, specifically including primary to tertiary amines such as trometamol, lidocaine, etc., amines containing basic heterocycles such as nicotinamide. These basic amine compounds may be used alone or in combination of two or more members. The concentration of the basic amine compound in the ophthalmic solution is preferably 0.01 to 15% (w/v), more preferably 0.1 to 10% (w/v), but not limited thereto.

The term "boric acids" used in the present invention means boric acid per se and its equivalent substances, which work as good buffers in the ophthalmic solutions. The equivalent substances to boric acid mean those compounds which release a borate ion on dissolution in water, including for example, boric anhydride ($B_2O_3$), pyroboric acid ($H_2B_4O_7$), as well as pharmacologically acceptable salts of boric acid, boric anhydride and pyroboric acid. Preferred examples include boric acid, boric anhydride, borax, and sodium borate decahydrate, particularly boric acid.

The above-mentioned boric acids may be used alone or in combination of two or more members.

The "ethylenediaminetetraacetic acids" mean ethylenediaminetetraacetic acid per se and its equivalent substances, which work as good stabilizers in the ophthalmic solutions. The equivalent substances to ethylenediaminetetraacetic acid mean those compounds which release an ethylenediaminetetraacetate ion on dissolution in water, including pharmacologically acceptable salts of ethylenediaminetetraacetic acid. Preferred examples include ethylenediaminetetraacetic acid, disodium ethylenediaminetetraacetate dihydrate, trisodium ethylenediaminetetraacetate trihydrate, and tetrasodium ethylenediaminetetraacetate tetrahydrate, more preferably disodium ethylenediaminetetraacetate dihydrate.

The above-mentioned ethylenediaminetetraacetic acids may be used alone or in combination of two or more members.

In the present invention, the intraocular pressure reducing effect of an ophthalmic solution containing the present compound can be enhanced by formulating "boric acids" and "ethylenediaminetetraacetic acids" in combination.

The present compound preferably employed includes:

4-(1-hydroxy-1-methylethyl)-2-propyl-1-{4-[2-(tetraz ol-5-yl)-phenyl]phenyl}methylimidazole-5-carboxylic acid; 2-ethoxy-1-[2'-(1H-tetrazol-5-yl)biphenyl-4-yl]methy 1]-1H-benzimidazole-7-carboxylic acid; or a pharmacologically acceptable salt or derivative thereof, but is not limited particularly thereto.

The concentration of the present compound in the ophthalmic solutions is preferably 0.01 to 15% (w/v), more preferably 0.1 to 10% (w/v), but not limited thereto.

The "pharmacologically acceptable salts" includes those prepared by reacting the present compound with a base. Such salts include metal salts, e.g., alkali metal salts such as sodium salt, potassium salt and lithium salt, alkaline earth metal salts such as calcium salt and magnesium salt, aluminum salt and iron salt; inorganic salts, e.g., ammonium salts; organic amine salts, e.g., t-octylamine salt, dibenzylamine salt, morpholine salt, glucosamine salt, phenylglycine alkylester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzylphenethylamine salt, piperazine salt, tetramethylammonium salt, tris(hydroxymethyl)aminomethane salt; and amino acid salts, e.g., glycine salt, lysine salt, arginine salt, ornithine salt, glutamic acid salt, aspartic acid salt. Preferably, alkali metal salt, more preferably sodium salt or potassium salt is used.

The "pharmacologically acceptable derivatives" of the present compound means those derived from the compounds having the chemical structure of the general formula (I), which has a hydroxyl group and/or carboxyl group, by modification of such groups. Such derivatives include "esters based on the hydroxyl group", "ethers based on the hydroxyl group", "esters based on the carboxy group" and "amides based on the carboxy group", that is, the compounds

in which each ester, ether or amide residue is a "general protective group" or "protective group which can be cleaved in vivo through a biological way such as hydrolysis".

The "general protective group" means one which can be cleaved by a chemical way such as hydrogenolysis, hydrolysis, electrolysis, photolysis, and the like.

The "general protective groups" associated with "esters based on the hydroxyl group" and "ethers based on the hydroxyl group" preferably are "aliphatic acyl groups" (preferably, lower aliphatic acyl groups of 1 to 6 carbons) including alkanoyl groups such as formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, eicosanoyl, heneicosanoyl, etc.; halogenoalkylcarbonyl groups such as chloroacetyl, dichloroacetyl, trichloroacetyl, trifluoroacetyl, etc.; and lower alkoxyalkylcarbonyl groups such as methoxyacetyl; and unsaturated alkylcarbonyl groups such as acryloyl, propioloyl, methacryloyl, crotonoyl, isocrotonoyl, (E)-2-methyl-2-butenoyl, etc.; "aromatic acyl groups" including arylcarbonyl groups such as benzoyl, α-naphthoyl, β-naphthoyl, etc.; halogenated arylcarbonyl groups such as 2-bromobenzoyl, 4-chlorobenzoyl, etc.; lower alkylated arylcarbonyl groups such as 2,4,6-trimethylbenzoyl, 4-toluoyl, etc.; lower alkoxylated arylcarbonyl groups such as 4-anisoyl; nitrated arylcarbonyl groups such as 4-nitrobenzoyl, 2-nitrobenzoyl, etc.; lower alkoxycarbonylated arylcarbonyl groups such as 2-(methoxycarbonyl)benzoyl; and arylated arylcarbonyl groups such as 4-phenylbenzoyl; "alkoxycarbonyl groups" including lower alkoxycarbonyl groups such as methoxycarbonyl, ethoxycarbonyl, propoxycarbonyl, butoxycarbonyl, s-butoxycarbonyl, t-butoxycarbonyl, isobutoxycarbonyl, etc.; and halogeno- or tri(lower alkyl)silyl-substituted lower alkoxycarbonyl groups such as 2,2,2-trichloroethoxycarbonyl, 2-trimethylsilylethoxycarbonyl, etc.; "tetrahydropyranyl or tetrahydrothiopyranyl groups" such as tetrahydropyran-2-yl, 3-bromotetrahydropyran-2-yl, 4-methoxytetrahydropyran-4-yl, tetrahydrothiopyran-2-yl, 4-methoxytetrahydrothiopyran-4-yl, etc.; "tetrahydrofuranyl or tetrahydrothiofuranyl groups" such as tetrahydrofuran-2-yl, tetrahydrothiofuran-2-yl, etc.; "silyl groups" including tri(lower alkyl)silyl groups such as trimethylsilyl, triethylsilyl, isopropyldimethylsilyl, t-butyldimethylsilyl, methyldiisopropylsilyl, methyldi-t-butylsilyl, triisopropylsilyl, etc.; and tri(lower alkyl)silyl groups substituted by 1 or 2 aryl groups such as diphenylmethylsilyl, diphenylbutylsilyl, diphenylisopropylsilyl, phenyldiisopropylsilyl, etc.; "alkoxymethyl groups" including lower alkoxymethyl groups such as methoxymethyl, 1,1-dimethyl-1-methoxymethyl, ethoxymethyl, propoxymethyl, isopropoxymethyl, butoxymethyl, t-butoxymethyl, etc.; lower alkoxylated lower alkoxymethyl groups such as 2-methoxyethoxymethyl; and halogeno-lower alkoxymethyl groups such as 2,2,2-trichloroethoxymethyl, bis(2-chloroethoxy)methyl, etc.; "substituted ethyl groups" including lower alkoxylated ethyl groups such as 1-ethoxyethyl, 1-(isopropoxy)ethyl, etc.; and halogenoethyl groups such as 2,2,2-trichloroethyl; "aralkyl groups" including lower alkyl groups substituted by 1 to 3 aryl groups such as benzyl, α-naphthylmethyl, β-naphthylmethyl, diphenylmethyl, triphenylmethyl, α-naphthyldiphenylmethyl, 9-anthrylmethyl, etc.; and lower alkyl groups substituted by 1 to 3 aryl groups in which the aryl ring is substituted by lower alkyl, lower alkoxy, nitro, halogen or cyano, such as 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl, 4-cyanobenzyl, etc.; "alkenyloxycarbonyl groups" such as vinyloxycarbonyl, allyloxycarbonyl, etc.; and "aralkyloxycarbonyl groups" in which the aryl ring may be substituted by 1 or 2 lower alkoxy or nitro groups, such as benzyloxycarbonyl, 4-methoxybenzyloxycarbonyl, 3,4-dimethoxybenzyloxycarbonyl, 2-nitrobenzyloxycarbonyl, 4-nitrobenzyloxycarbonyl, etc.

The "general protective groups" associated with "esters based on the carboxy group" include "lower alkyl groups" such as methyl, ethyl, propyl, isopropyl, n-butyl, isobutyl, s-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, etc.; "lower alkenyl groups" such as vinyl, 2-propenyl, 1-methyl-2-propenyl, 2-methyl-2-propenyl, 2-ethyl-2-propenyl, 2-butenyl, 1-methyl-2-butenyl, 2-methyl-2-butenyl, 1-ethyl-2-butenyl, 3-butenyl, 1-methyl-3-butenyl, 2-methyl-3-butenyl, 1-ethyl-3-butenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 2-hexenyl, 3-hexenyl, 4-hexenyl, 5-hexenyl, etc.; "lower alkynyl groups" such as ethynyl, 2-propynyl, 1-methyl-2-propynyl, 2-butynyl, 1-methyl-2-butynyl, 1-ethyl-2-butynyl, 3-butynyl, 1-methyl-3-butynyl, 2-methyl-3-butynyl, 1-ethyl-3-butynyl, 2-pentynyl, 1-methyl-2-pentynyl, 3-pentynyl, 1-methyl-3-pentynyl, 2-methyl-3-pentynyl, 4-pentynyl, 1-methyl-4-pentynyl, 2-methyl-4-pentynyl, 2-hexynyl, 3-hexynyl, 4-hexynyl, 5-hexynyl, etc.; "halogeno-lower alkyl" such as trifluoromethyl, trichloromethyl, difluoromethyl, dichloromethyl, dibromomethyl, fluoromethyl, 2,2,2-trichloroethyl, 2,2,2-trifluoroethyl, 2-bromoethyl, 2-chloroethyl, 2-fluoroethyl, 2,2-dibromoethyl, etc.; hydroxy-"lower alkyl groups" such as 2-hydroxyethyl, 2,3-dihydroxypropyl, 3-hydroxypropyl, 3,4-dihydroxybutyl, 4-hydroxybutyl, etc.; "lower aliphatic acyl"-"lower alkyl groups" such as acetylmethyl; the above-mentioned "aralkyl groups"; and the above-mentioned "silyl groups".

The term "protective group which can be cleaved in vivo through a biological way such as hydrolysis" means a protective group which can be cleaved by a biological process such as hydrolysis in the human body to give a free

acid or its salt. Such a derivative can be confirmed by intravenously injecting it to an experimental animal, for example, rat or mouse, followed by examination of the animal's body fluid and detection of the original compound or its pharmacologically acceptable salt. The "protective group which can be cleaved in vivo through a biological way such as hydrolysis" associated with "esters based on the hydroxyl group" and "ethers based on the hydroxyl group" preferably are "carbonyloxyalkyl groups" such as 1-(acyloxy) "lower alkyl groups" including 1-("lower aliphatic acyl"oxy) "lower alkyl groups" such as formyloxymethyl, acetoxymethyl, dimethylaminoacetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isoveleryloxypropyl, 1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl, 1-pivaloyloxyhexyl, etc.; 1-("cycloalkyl" carbonyloxy) "lower alkyl groups" such as cyclopentylcarbonyloxymethyl, cyclohexylcarbonyloxymethyl, 1-cyclopentylcarbonyloxyethyl, 1-cyclohexylcarbonyloxyethyl, 1-cyclopentylcarbonyloxypropyl, 1-cyclohexylcarbonyloxypropyl, 1-cyclopentylcarbonyloxybutyl, 1-cyclohexylcarbonyloxybutyl, etc.; and 1-("aromatic acyl"oxy) "lower alkyl groups" such as benzoyloxymethyl; (lower alkoxycarbonyloxy)alkyl groups such as methoxycarbonyloxymethyl, ethoxycarbonyloxymethyl, propoxycarbonyloxymethyl, isopropoxycarbonyloxymethyl, butoxycarbonyloxymethyl, isobutoxycarbonyloxymethyl, pentyloxycarbonyloxymethyl, hexyloxycarbonyloxymethyl, cyclohexyloxycarbonyloxymethyl, cyclohexyloxycarbonyloxy(cyclohexyl)methyl, 1-(methoxycarbonyloxy)ethyl, 1-(ethoxycarbonyloxy)ethyl, 1-(propoxycarbonyloxy)ethyl, 1-(isopropoxycarbonyloxy)ethyl, 1-(butoxycarbonyloxy)ethyl, 1-(isobutoxycarbonyloxy)ethyl, 1-(t-butoxycarbonyloxy)ethyl, 1-(pentyloxycarbonyloxy)ethyl, 1-(hexyloxycarbonyloxy)ethyl, 1-(cyclopentyloxycarbonyloxy)ethyl, 1-(cyclopentyloxycarbonyloxy)propyl, 1-(cyclohexyloxycarbonyloxy)propyl, 1-(cyclopentyloxycarbonyloxy)butyl, 1-(cyalohexyloxycarbonyloxy)butyl, 1-(cyclohexyloxycarbonyloxy)ethyl, 1-(ethoxycarbonyloxy)propyl, 1-(methoxycarbonyloxy)propyl, 1-(ethoxycarbonyloxy)propyl, 1-(propoxycarbonyloxy)propyl, 1-(isopropoxycarbonyloxy)propyl, 1-(butoxycarbonyloxy)propyl, 1-(isobutoxycarbonyloxy)propyl, 1-(pentyloxycarbonyloxy) propyl, 1-(hexyloxycarbonyloxy)propyl, 1-(methoxycarbonyloxy)butyl, 1-(ethoxycarbonyloxy)butyl, 1-(propoxycarbonyloxy)butyl, 1-(isopropoxycarbonyloxy)butyl, 1-(butoxycarbonyloxy)butyl, 1-(isobutoxycarbonyloxy)butyl, 1-(methoxycarbonyloxy)pentyl, 1-(ethoxycarbonyloxy)pentyl, 1-(methoxycarbonyloxy)hexyl, 1-(ethoxycarbonyloxy)hexyl, etc.; and oxodioxolenylmethyl groups such as (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl)methyl, [5-(4-fluorophenyl)-2-oxo-1,3-dioxolen-4-yl] methyl, [5-(4-chlorophenyl)-2-oxo-1,3-dioxolen-4-yl]methyl, (2-oxo-1,3-dioxolen-4-yl)methyl, (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl, (5-butyl-2-oxo-1,3-dioxolen-4-yl)methyl, etc.; "phthalidyl groups" such as phthalidyl, dimethylphthalidyl, dimethoxyphthalidyl, etc.; the above-mentioned "lower aliphatic acyl groups"; the above-mentioned "aromatic acyl groups"; "succinic acid half-ester salt residues"; "phosphoric acid ester salt residues"; "ester-forming residues of amino acids"; carbamoyl groups; carbamoyl groups substituted by 1 or 2 lower alkyl groups; and "1-(acyloxy) alkyloxycarbonyl groups" such as pivaloyloxymethyloxycarbonyl. Preferred example includes "carbonyloxyalkyl groups".

On the other hand, "protective groups which can be cleaved in vivo through a biological way such as hydrolysis" associated with "esters based on the carboxy group" are preferably "alkoxy lower alkyl groups" including lower alkoxy-lower alkyl groups such as methoxyethyl, 1-ethoxyethyl, 1-methyl-1-methoxyethyl, 1-(isopropoxy)ethyl, 2-methoxyethyl, 2-ethoxyethyl, 1,1-dimethyl-1-methoxyethyl, ethoxymethyl, n-propoxymethyl, isopropoxymethyl, n-butoxymethyl, t-butoxymethyl, etc. ; lower alkoxylated lower alkoxy-lower alkyl groups such as 2-methoxyethoxymethyl; "aryl"oxy "lower alkyl groups" such as phenoxymethyl; halogenated lower alkoxy-lower alkyl groups such as 2,2,2-trichloroethoxymethyl, bis(2-chloroethoxy)methyl, etc.; ""lower alkoxy"carbonyl "lower alkyl groups"" such as methoxycarbonylmethyl; cyano "lower alkyl groups" such as cyanomethyl, 2-cyanoethyl, etc.; ""lower alkyl" thiomethyl groups" such as methylthiomethyl, ethylthiomethyl, etc.; ""aryl"thiomethyl groups" such as phenylthiomethyl, naphthylthiomethyl, etc.; "optionally halogenated "lower alkyl"sulfonyl "lower alkyl groups"" such as 2-methanesulfonylethyl, 2-trifluoromethanesulfonylethyl, etc.; ""aryl"sulfonyl "lower alkyl groups"" such as 2-benzenesulfonylethyl, 2-toluenesulfonylethyl, etc.; the above-mentioned "1-(acyloxy) "lower alkyl groups""; the above-mentioned "phthalidyl groups"; the above-mentioned "lower alkyl groups"; "carboxyalkyl groups" such as carboxymethyl; and "amide-forming residues of amino acids" such as phenylalanine.

The term "dispersion in a state of molecule" indicates that in the case where the present compound is formulated in an ophthalmic solution, the chemical shift of each proton appears at a higher magnetic field (upfield) compared with the shift in an aqueous solution of the present compound. More specifically, it indicates a state in which the protons on the phenyl ring of the present compounds are shifted by 0.01ppm or more, preferably 0.02ppm or more, to the higher upfield position. Details will be described in Examples below. The state of molecular dispersion can be confirmed by the fact that the particle size of the present compound prepared in an ophthalmic solution becomes about 1/2 or less of that in an aqueous solution, indicating it being clearly dispersed almost in a state of the molecular form in the

solution.

The ophthalmic solutions of the present invention may optionally be prepared in combination with additives, for example, buffer, stabilizer, isotonic agent, pH adjustor, solubilizing agent, thickener, dispersant, and so on.

The buffer is exemplified by the above-mentioned boric acids, as well as phosphoric acids, acetic acids, carbonic acids, citric acids, and the like. The phosphoric acids, acetic acids, carbonic acids, and citric acids include phosphoric acid, acetic acid, carbonic acid, citric acid, per se, and their equivalent substances. The equivalent substances to phosphoric acid, acetic acid, carbonic acid, and citric acid mean compounds which generate phosphate ion, acetate ion, carbonate ion, and citrate ion on dissolution in water. Preferred ones are boric acids, particularly, boric acid, boric anhydride, borax and sodium borate decahydrate.

The stabilizer is exemplified by the above-mentioned ethylenediaminetetraacetic acids, as well as dibutylhydroxytoluene, sodium hydrogen sulfite, and the like. Preferred are ethylenediaminetetraacetic acids, and particularly preferred are ethylenediaminetetraacetic acid, disodium ethylenediaminetetraacetate dihydrate, trisodium ethylenediaminetetraacetate trihydrate, and tetrasodium ethylenediaminetetraacetate tetrahydrate.

Since the combined use of boric acids and ethylenediaminetetraacetic acids enhances the intraocular pressure reducing effect of the ophthalmic solutions, when a buffer and a stabilizer are added, it is appropriate to add boric acids as a buffer and ethylenediaminetetraacetic acids as a stabilizer in combination.

The isotonic agent includes, for example, glycerin, propylene glycol, sodium chloride, potassium chloride, sorbitol, mannitol, and the like.

The pH adjustor includes, for example, hydrochloric acid, citric acid, phosphoric acid, acetic acid, sodium hydroxide, potassium hydroxide, boric acid, borax, sodium carbonate, sodium bicarbonate, and the like.

Thesolubilizing agentincludes,for example, polysorbate 80, polyoxyethylene hardened castor oil 60, macrogol 4000, polyethylene glycol, propylene glycol, and the like.

The thickener and dispersant include, for example, macromolecules of cellulose type such as hydroxypropylmethylcellulose, hydroxypropylcellulose, etc., sodium alginate, polyvinyl alcohol, carboxyvinylpolymer, polyvinyl pyrrolidone, and the like.

The ophthalmic solutions of the present invention may preferably be adjusted at pH 5.0 to 9.0 and at approximately 1.0 of osmotic pressure ratio. The dose of the ophthalmic solution may be selected depending on the condition, age, and the formulation, and practically may be administered to eyes in a concentration of 0.01 to 15%(w/v) of the present compound one to several times a day.

Brief Description of Drawings

Fig. 1 shows variables of the chemical shift in Example 5 in comparison with those in Comparative Example 2.
Fig. 2 shows variables of the chemical shift in Examples 6 and 7 and Comparative Example 4 in comparison with those in Comparative Example 3.

Best Mode for Carrying Out the Invention

The following examples serve to illustrate specifically the invention for the purpose of better understanding but are not intended to limit the scope of the invention.

(1) Preparation Example

The followings show typical prescriptions of ophthalmic solutions used in the invention. Compound A means 4-(1-hydroxy-1-methylethyl)-2-propyl-1-{4-[2-(tetrazol-5-y l)phenyl]phenyl}methylimidazole-5-carboxylic acid, having the following structural formula.

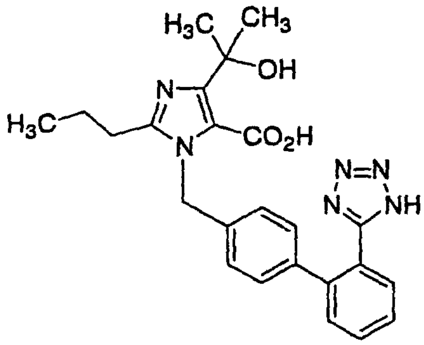

Compound A

| Prescription 1 (in 100ml) | |
|---|---|
| Compound A | 4 g |
| Benzalkonium chloride | 0.01 g |
| Trometamol | 2 g |
| Sodium chloride | 0.28 g |
| Sodium dihydrogen phosphate dihydrate | proper amount |
| Sodium hydroxide | proper amount |
| Diluted hydrochloric acid | proper amount |
| Sterilized pure water | proper amount |

The sort of the compound and the mixing ratio of the additives may optionally be changed to give a desired ophthalmic solution.

| Prescription 2 (in 100ml) | |
|---|---|
| Compound A | 3 g |
| Benzalkonium chloride | 0.005 g |
| Trometamol | 2 g |
| Boric acid | 0.5 g |
| Disodium ethylenediaminetetraacetate dihydrate | 0.005 g |
| Sodium chloride | 0.2 g |
| Sodium dihydrogen phosphate dihydrate | proper amount |
| Sodium hydroxide | proper amount |
| Diluted hydrochloric acid | proper amount |
| Sterilized pure water | proper amount |

The sort of the compound and the mixing ratio of the additives may optionally be changed to give a desired ophthalmic solution.

| Prescription 3 (in 100ml) | |
|---|---|
| Compound A | 3 g |
| Cetrimide | 0.005 g |
| Trometamol | 2 g |
| Boric acid | 0.5 g |
| Disodium ethylenediaminetetraacetate dihydrate | 0.005 g |
| Sodium chloride | 0.2 g |
| Sodium dihydrogen phosphate dihydrate | proper amount |
| Sodium hydroxide | proper amount |
| Diluted hydrochloric acid | proper amount |

(continued)

| Prescription 3 (in 100ml) | |
|---|---|
| Sterilized pure water | proper amount |

The sort of the compound and the mixing ratio of the additives may optionally be changed to give a desired ophthalmic solution.

(2) Preservation effectiveness test

In order to examine the antiseptic effect of the ophthalmic solutions of the present invention, the following preservation effectiveness test was conducted.

[Preparation of ophthalmic solutions]

Example 1

In 75ml of purified water was dissolved 2 g of trometamol, ethylenediaminetetraacetic acid (proper amount) and borax (proper amount). To this solution was added 4 g of Compound A and then, after dissolution, 0.004 g of benzalkonium chloride. The solution was adjusted at pH 7.0 with 1 mol/L sodium hydroxide solution, and then made up to 100 ml in total with addition of purified water. The solution was then filtered through a membrane filter (manufactured by MILLIPORE)[hydrophilic Durapore (material: hydrophilic polyvinylidene difluoride), 0.22μm] in a sterilized condition to give an ophthalmic solution.

Example 2

An ophthalmic solution was prepared in the same manner as in Example 1, except that cetrimide was used in place of benzalkonium chloride.

Example 3

An ophthalmic solution was prepared in the same manner as in Example 1, except that lidocaine was used in place of trometamol.

Example 4

An ophthalmic solution was prepared in the same manner as in Example 1, except that nicotinamide was used in place of trometamol.

Comparative Example 1

An ophthalmic solution was prepared in the same manner as in Example 1, except that no trometamol was added thereto.

[Test Method]

The preservation effectiveness test was carried out according to the Japanese Pharmacopoeia XIV. E. coli, P. aeruginosa, S. aureus, and A. niger were used as test microorganisms. Viable microbe cell number was measured 6 hours (for bacteria), 24 hours (for bacteria), and 7 days (for fungus) after incubation, and the survival rate of the microorganisms was calculated according to the following equation. Tables 1 and 2 show the results.

Survival rate (%) = Viable microbe cell number /Initial

number X 100

Table 1

| | | Example 1 | Example 2 | Cmp.Ex.1 |
|---|---|---|---|---|
| Compound A (g) | | 4 | 4 | 4 |
| Benzalkonium chloride (g) | | 0.004 | | 0.004 |
| Cetrimide (g) | | | 0.004 | |
| Trometamol (g) | | 2 | 2 | |
| Borax (g) | | proper amount | proper amount | proper amount |
| EDTA* (g) | | proper amount | proper amount | proper amount |
| pH | | 7 | 7 | 7 |
| E.coli (%) | 6 hr after | $4.3x10^{-4}$ or less | $9.5x10^{-4}$ | $2.2x10^{-3}$ |
| | 24 hr after | $4.3x10^{-4}$ or less | $1.0x10^{-4}$ or less | $2.9x10^{-2}$ |
| P.aeruginosa (%) | 6 hr after | $3.3x10^{-4}$ or less | $2.3x10^{-3}$ or less | $9.7x10^{-1}$ |
| | 24 hr after | $3.3x10^{-4}$ orless | $2.3x10^{-3}$ or less | $4.0x10^{-1}$ |
| S.aureus (%) | 6 hr after | $6.3x10^{-4}$ | $5.6x10^{-3}$ | $1.0x10^{-2}$ |
| | 24 hr after | $3.1x10^{-4}$ or less | $1.4x10^{-3}$ or less | $3.1x10^{-4}$ or less |
| A.niger (%) | 7 days after | 1.6 | $1.6x10^{-1}$ | 7.7 |

*EDTA: Ethylenediaminetetraacetic acid

Table 2

| | | Example 3 | Example 4 | Cmp.Ex.1 |
|---|---|---|---|---|
| Compound A (g) | | 4 | 4 | 4 |
| Benzalkonium chloride (g) | | 0.004 | 0.004 | 0.004 |
| Lidocaine (g) | | 2 | | |
| Nicotinamide (g) | | | 2 | |
| Borax (g) | | proper amount | proper amount | proper amount |
| EDTA* (g) | | proper amount | proper amount | proper amount |
| pH | | 7 | 7 | 7 |
| E.coli (%) | 6 hr after | $4.7x10^{-3}$ | $5.9x10^{-1}$ | 1.6 |
| | 24 hr after | $9.3x10^{-4}$ | $1.7x10^{-2}$ | $6.9x10^{-1}$ |
| P.aeruginosa (%) | 6 hr after | $1.3x10^{-4}$ or less | $6.5x10^{-3}$ | $1.0x10^{-1}$ |
| | 24 hr after | $1.3x10^{-4}$ or less | $1.3x10^{-4}$ or less | $2.3x10^{-3}$ |
| S.aureus (%) | 6 hr after | undone | undone | undone |
| | 24 hr after | undone | undone | undone |
| A.niger (%) | 7 days after | 38 | $1.0x10^{-3}$ | 22 |

*EDTA: Ethylenediaminetetraacetic acid

[Stability Test]

The ophthalmic solutions of Examples 1 to 4 were preserved at 20°C under a humidity of 40% for a period of 4 months; there was no change in appearance, pH, and the residual rate of Compound A and no occurrence of degradation products in the solutions. Though the ophthalmic solution containing a low concentration (approximately 0.5%) of Compound A and a cationic antiseptic agent became turbid, the turbidity was improved by adding a basic amine compound.

(3) Measurement of the chemical shift by NMR

[Preparation of a sample for use in NMR measurement]

Example 5

To 80 mL of heavy water was added 1 g of Compound A and 2 g of trometamol. In this solution was dissolved 1 mol/L sodium hydroxide solution (NaOD), which was then adjusted to pH 7.0 with 1 mol/L hydrochloric acid (DC1) and made up to 100 mL in total with addition of heavy water. The solution was then filtered through a membrane filter (MILLIPORE) in a sterile condition to give a sample solution.

Comparative Example 2

A sample solution for comparison was prepared in the same manner as in Example 5, except that no trometamol was added.

Example 6

In 80 mL of heavy water was dissolved 4 g of Compound A and 2 g of trometamol. The solution was adjusted to pH 7.0 with 1 mol/L sodium hydroxide solution (NaOD), which was made up to 100 mL in total with addition of heavy water. The solution was then filtered through a membrane filter (MILLIPORE) in a sterile condition to give a sample solution.

Comparative Example 3

A sample solution for comparison was prepared in the same manner as in Example 6, except that no trometamol was added.

Example 7

In 80 mL of heavy water was dissolved 4 g of Compound A, 2 g of trometamol, 0.004 g of benzalkonium chloride, ethylenediaminetetraacetic acid (proper amount) and borax (proper amount). The solution was adjusted to pH 7.0 with 1 mol/L sodium hydroxide solution (NaOD), which was made up to 100 mL in total with addition of heavy water. The solution was then filtered through a membrane filter (MILLIPORE) in a sterile condition to give a sample solution.

Comparative Example 4

A sample solution for comparison was prepared in the same manner as in Example 7, except that no trometamol was added.

[Test Method]

$^1$H-NMR spectra were measured using an NMR apparatus (JEOL ECP-500) (measured at 35°C; internal standard HDO; solvent $D_2O$). The variables of the chemical shift (unit: ppm) of Compound A in Example 5 versus that (standard) in Comparative Example 2 are shown in Table 3 and Fig.1. The variables of the chemical shift of Compound A in Examples 6, 7 and Comparative Example 4 versus that (standard) in Comparative Example 3 are shown in Table 4 and Fig.2. In this connection, the symbols a to k in Tables and Figs correspond to the variables of the chemical shift of the hydrogen atoms attached to the carbons a to k as shown in the following structural formula.

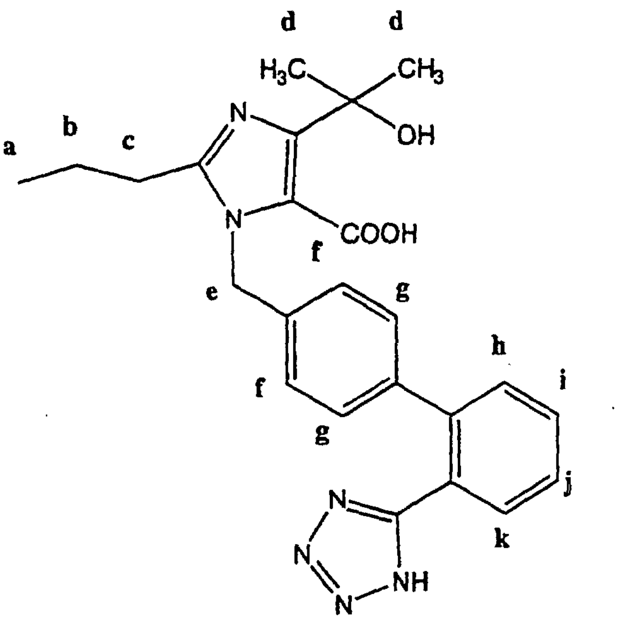

Table 3

| | | Example 5 | Cmp. Example 2 |
|---|---|---|---|
| Compound A (g) | | 1 | 1 |
| Trometamol (g) | | 2 | |
| pH | | 7 | 7 |
| Chemical shift Δ (ppm) | a | -0.028 | 0.000 |
| | b | -0.027 | 0.000 |
| | c | -0.036 | 0.000 |
| | d | -0.021 | 0.000 |
| | e | -0.025 | 0.000 |
| | f | -0.034 | 0.000 |
| | g | -0.034 | 0.000 |
| | h | -0.045 | 0.000 |
| | i | -0.031 | 0.000 |
| | j | -0.030 | 0.000 |
| | k | -0.023 | 0.000 |
| Mean value of a to k | | -0.030 | 0.000 |

Table 4

| | Example 6 | Example 7 | Cpm Exm 3 | Cpm Exm 4 |
|---|---|---|---|---|
| Compound A (g) | 4 | 4 | 4 | 4 |
| Trometamol (g) | 2 | 2 | | |
| Benzalkonium Chloride (g) | | 0.005 | | 0.005 |

Table 4 (continued)

| | | Example 6 | Example 7 | Cpm Exm 3 | Cpm Exm 4 |
|---|---|---|---|---|---|
| Borax (g) | | | prop.amt | | prop.amt |
| EDTA* (g) | | | prop.amt | | prop.amt |
| pH | | 7 | 7 | 7 | 7 |
| Chemical shift Δ (ppm) | a | -0.032 | -0.032 | 0.000 | -0.003 |
| | b | -0.033 | -0.033 | 0.000 | -0.004 |
| | c | -0.026 | -0.031 | 0.000 | -0.001 |
| | d | -0.027 | -0.028 | 0.000 | -0.001 |
| | e | -0.029 | -0.030 | 0.000 | -0.002 |
| | f | -0.029 | -0.032 | 0.000 | -0.001 |
| | g | -0.030 | -0.031 | 0.000 | -0.002 |
| | h | -0.035 | -0.036 | 0.000 | -0.005 |
| | i | -0.035 | -0.036 | 0.000 | -0.002 |
| | j | -0.036 | -0.037 | 0.000 | -0.003 |
| | k | -0.032 | -0.032 | 0.000 | -0.001 |
| Mean value of a to k | | -0.031 | -0.033 | 0.000 | -0.002 |

*EDTA: Ethylenediaminetetraacetic acid

(4) Measurement of the particle size

[Method of Measurement]

The particle size of Compound A in Example 6 and Comparative Example 3 (wherein water was used in place of heavy water, and NaOH in place of NaOD) was measured using a particle size measurement apparatus [NICOMP: Submicron Particle Sizer Model 370] at a wavelength of 514.5 nm and a temperature of 23°C (measuring time: 10 min).

[Result of Measurement]

The mean particle size of Compound A in Example 6 was reduced to 1/2 or less in comparison with that of Comparative Example 3.

Industrial Applicability

As apparent from Tables 1 and 2, the antimicrobial effect of the ophthalmic solutions containing Compound A and a cationic antiseptic agent (e.g., benzalkonium chloride, cetrimide) to bacteria and fungi is markedly increased by adding a basic amine compound to the solutions. Therefore, an ophthalmic solution comprising a compound having the chemical structure of the general formula (I) or a pharmaceutically acceptable salt or derivative thereof, a cationic antiseptic agent and a basic amine compound exhibits a highly potent preservative effect against a variety of microorganisms such as bacteria and fungi. From the results as shown in Tables 3 and 4 and Figs. 1 and 2, it is found that in the ophthalmic solutions of the present invention the chemical shift of each proton of Compound A is shifted to the higher magnetic field and the mean particle size of micell is reduced to 1/2 or less.

## Claims

**1.** An ophthalmic solution comprising a compound having the chemical structure of the following general formula (I) or a pharmaceutically acceptable salt or derivative thereof , a cationic antiseptic agent and a basic amine compound:

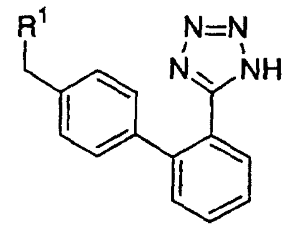

(I)

[wherein $R^1$ represents a group of the following structural formula (Ia), (Ib), (Ic), (Id), (Ie) or (If)]

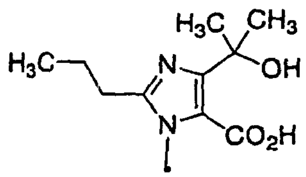

(Ia)

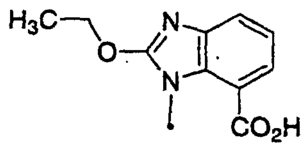

(Ib)

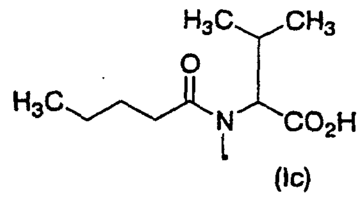

(Ic)

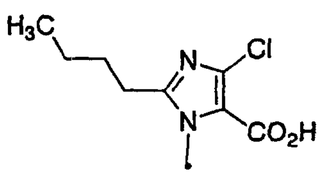

(Id)

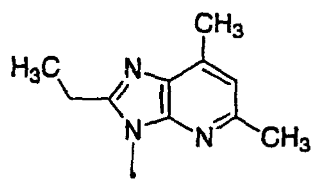

(Ie)

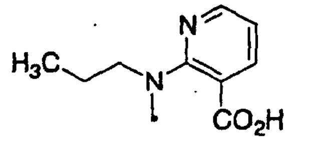

(If)

**2.** The ophthalmic solution of Claim 1, wherein $R^1$ is a group of the structural formula (Ia), (Ib) or (Ic).

**3.** The ophthalmic solution of Claim 1, wherein the compound having the chemical structure of the general formula (I) is selected from:

4-(1-hydroxy-1-methylethyl)-2-propyl-1-{4-[2-(tetraz ol-5-yl)phenyl]phenyl}methylimidazole-5-carboxylic acid and
2-ethoxy-1-[2'-(1H-tetrazol-5-yl)biphenyl-4-yl]methy l]-1H-benzimidazole-7-carboxylic acid.

**4.** The ophthalmic solution of any one of Claims 1 to 3, wherein the cationic antiseptic agent is a quaternary ammonium salt.

**5.** The ophthalmic solution of Claim 4, wherein the quaternary ammonium salt is benzalkonium chloride or cetrimide.

**6.** The ophthalmic solution of any one of Claims 1 to 5, wherein the basic amine compound is at least one selected from trometamol, lidocaine and nicotinamide.

**7.** The ophthalmic solution of any one of Claims 1 to 6, additionally containing boric acids.

**8.** The ophthalmic solution of any one of Claims 1 to 6, additionally containing ethylenediaminetetraacetic acids.

**9.** The ophthalmic solution of any one of Claims 1 to 6, additionally containing boric acids and ethylenediaminetetraacetic acids.

**10.** An ophthalmic solution containing as an active ingredient a compound having the chemical structure of the general formula (I) or a pharmaceutically acceptable salt or derivative thereof as described in Claim 1, wherein the active ingredient is dispersed in a molecular state in a vehicle for ophthalmic solutions.

**11.** The ophthalmic solution of Claims 10, wherein a cationic antiseptic agent is formulated as an antiseptic.

**12.** The ophthalmic solution of Claims 10, wherein the active ingredient is selected from:

4-(1-hydroxy-1-methylethyl)-2-propyl-1-{4-[2-(tetraz ol-5-yl)phenyl]phenyl}methylimidazole-5-carboxylic acid and
2-ethoxy-1-[2'-(1H-tetrazol-5-yl)biphenyl-4-yl]methy 1]-1H-benzimidazole-7-carboxylic acid,

or is a pharmacologically acceptable salt thereof.

Fig. 1

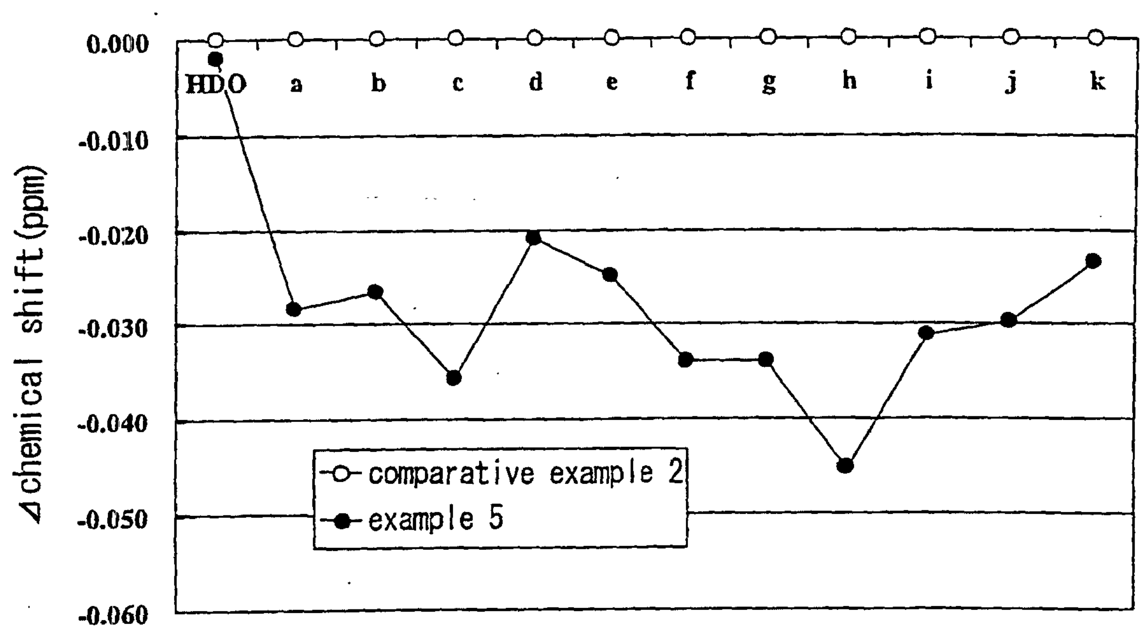
0.000
-0.010
-0.020
-0.030
-0.040
-0.050
-0.060
Δchemical shift (ppm)
HDO
a
b
c
d
e
f
g
h
i
j
k
comparative example 2
example 5

Fig. 2

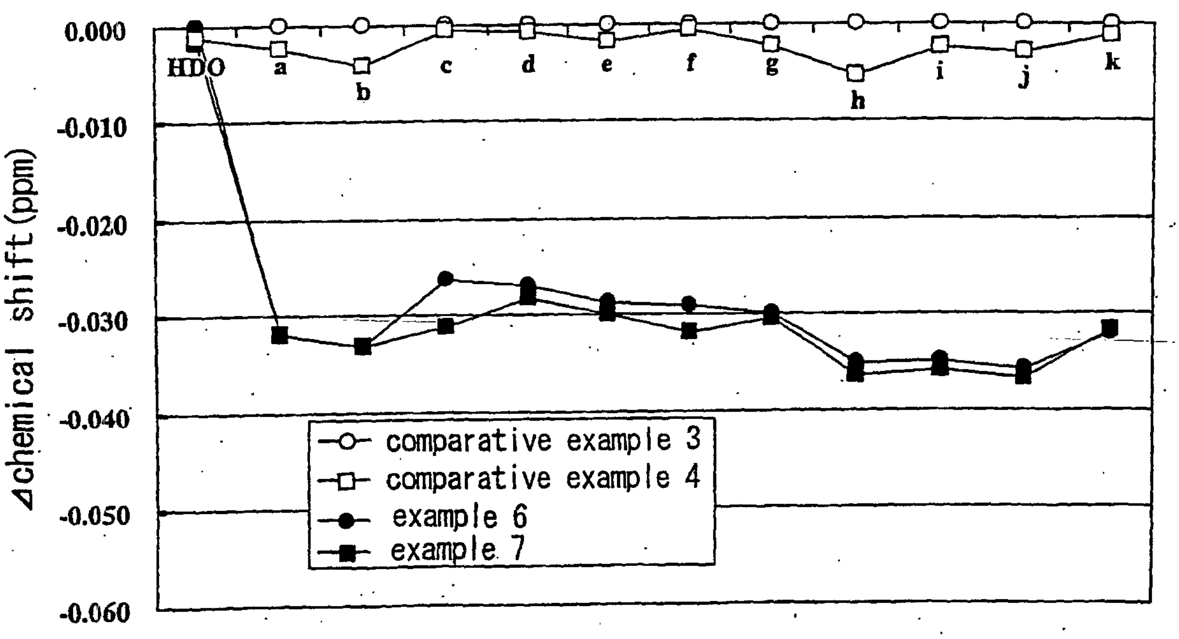
0.000
-0.010
-0.020
-0.030
-0.040
-0.050
-0.060
Δchemical shift (ppm)
HDO
a
b
c
d
e
f
g
h
i
j
k
comparative example 3
comparative example 4
example 6
example 7

## INTERNATIONAL SEARCH REPORT

International application No.
PCT/JP03/02771

A. CLASSIFICATION OF SUBJECT MATTER
Int.Cl[7] A61K31/41, 31/4178, 31/4184, 31/437, 31/4439, 47/18, A61P27/02, A07D257/04, 401/10, 403/10, 471/04

According to International Patent Classification (IPC) or to both national classification and IPC

B. FIELDS SEARCHED

Minimum documentation searched (classification system followed by classification symbols)
Int.Cl[7] A61K31/41, 31/4178, 31/4184, 31/437, 31/4439, 47/18, A61P27/02, A07D257/04, 401/10, 403/10, 471/04

Documentation searched other than minimum documentation to the extent that such documents are included in the fields searched

| | | | |
|---|---|---|---|
| Jitsuyo Shinan Koho | 1926-1996 | Toroku Jitsuyo Shinan Koho | 1994-1996 |
| Kokai Jitsuyo Shinan Koho | 1971-1992 | Jitsuyo Shinan Toroku Koho | 1996-2003 |

Electronic data base consulted during the international search (name of data base and, where practicable, search terms used)
CA(STN), BIOSIS(STN), MEDLINE(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| Y | JP 2001-55329 A (Sankyo Co., Ltd.), 27 February, 2001 (27.02.01), Full text & EP 1186302 A1 | 1-12 |
| Y | JP 2001-172183 A (Wakamoto Pharmaceutical Co., Ltd.), 26 June, 2001 (26.06.01), Claims; Par. Nos. [0018], [0020]; examples (Family: none) | 1-12 |
| Y | JP 11-302162 A (Kissei Pharmaceutical Co., Ltd.), 02 November, 1999 (02.11.99), Claims; Par. Nos. [0013], [0016], [0017]; examples (Family: none) | 1-12 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 June, 2003 (16.06.03) | 01 July, 2003 (01.07.03) |
| Name and mailing address of the ISA/ Japanese Patent Office | Authorized officer |
| Facsimile No. | Telephone No. |

Form PCT/ISA/210 (second sheet) (July 1998)

## INTERNATIONAL SEARCH REPORT

International application No.
PCT/JP03/02771

C (Continuation). DOCUMENTS CONSIDERED TO BE RELEVANT

| Category* | Citation of document, with indication, where appropriate, of the relevant passages | Relevant to claim No. |
|---|---|---|
| Y | US 4829088 A (Dispersa A.-G.), 09 May, 1989 (09.05.89), Claims; column 3, lines 42 to 52; column 4, line 16; examples & JP 62-242617 A & EP 242328 A2 | 1-12 |

Form PCT/ISA/210 (continuation of second sheet) (July 1998)